Europäisches Patentamt

European Patent Office

Office européen des brevets

(19)

(11) Veröffentlichungsnummer: **0 303 045**

**A1**

(12) **EUROPÄISCHE PATENTANMELDUNG**

(21) Anmeldenummer: 88110742.9

(22) Anmeldetag: 06.07.88

(51) Int. Cl.⁴: **A61K 9/00 , A61B 5/06**

Die Bezeichnung der Erfindung wurde geändert (Richtlinien für die Prüfung im EPA, A-III, 7.3).

(30) Priorität: 15.07.87 DE 3723310

(43) Veröffentlichungstag der Anmeldung:
15.02.89 Patentblatt 89/07

(84) Benannte Vertragsstaaten:
AT BE CH DE ES FR GB GR IT LI LU NL SE

(71) Anmelder: **Urquhart, John, Dr.**
**975 Hamilton Avenue**
**Palo-Alto California 94301(US)**

(72) Erfinder: **Urquhart, John, Dr.**
**975 Hamilton Avenue**
**Palo-Alto California 94301(US)**

(74) Vertreter: **Hafner, Dieter, Dr.rer.nat.,**
**Dipl.-Phys.**
**Ostendstrasse 132**
**D-8500 Nürnberg 30(DE)**

(54) **Magnetische Werkstoffe enthaltende pharmazeutische Präparate.**

(57) Einem pharmazeutischen Präparat ist wenigstens ein magnetischer Werkstoff zugesetzt, der auf ein von außen einwirkendes Magnetfeld einer elektronischen Überwachungsvorrichtung reagierfähig ist, um ein durch diese Überwachungsvorrichtung selektiv detektierbares Signal zur Lieferung einer eindeutigen Information abzugeben, ob das Präparat von einem Patienten eingenommen ist oder nicht und/oder zur Lokalisierung des Präparats bzw. des magnetischen Werkstoffes im Bereich des Gastro-Intestinal-Traktes des Patienten.

EP 0 303 045 A1

## Pharmazeutisches Präparat und Verfahren zu seiner Herstellung.

Die Erfindung betrifft ein pharmazeutisches Präparat (Arzneimittel), insbesondere zur oralen oder analen Verabreichung.

Der Stand der Technik:

In der medizinischen Literatur hat man sich bereits ausgiebig mit der Problematik beschäftigt, die sich bezüglich der Bereitwilligkeit, Fügsamkeit und Gewissenhaftigkeit der Patienten ergibt, die ihnen vom behandelnden Arzt verordneten Medikamente oder Arzneimittel in tatsächlicher Übereinstimmung mit der verordneten Dosierung und zu den vorgeschriebenen Zeitpunkten einzunehmen.

Aus der vorgenannten Literatur läßt sich im wesentlichen folgendes entnehmen:

1. Es gibt eine bedeutende Minorität von Patienten, denen bei der "Selbstverwaltung" der verordneten Arzneimittel große Irrtümer unterlaufen.

2. Diese Irrtümer treten mehr oder weniger unabhängig von der Ernsthaftigkeit oder Heftigkeit der jeweiligen Erkrankung auf.

3. Die Patienten verhalten sich erfahrungsgemäß im allgemeinen so, daß sie die hinsichtlich der Dosierung bzw. der Arzneimitteleinnahme überhaupt gemachten Fehler vor ihren Ärzten verbergen oder verheimlichen; hierbei ist auch festzustellen, daß aufgrund des Verhaltens ärztlicherseits und des diagnostischen Accumens die meisten Patienten tatsächlich darin erfolgreich sind, Art und Ausmaß der ihnen unterlaufenen Dosierungsirrtümer oder -fehler vor den behandelnden Ärzten verheimlichen zu können.

4. Der im allgemeinen am häufigsten vorkommende Fehler hinsichtlich der Arzneimittel-Dosierung besteht in der Unterlassung oder dem Versäumnis der Einnahme der verordneten Arzneimittel.

5. Als weniger häufig und wahrscheinlich an die zweite Stelle tretend ist im allgemeinen ein Dosierungsfehler zu bezeichnen, welcher in dem Vergessen besteht, daß die vorgeschriebene Dosis bereits eingenommen worden ist, so daß diese nach Ablauf von etwa einer Stunde oder einer längeren Zeitspanne erneut eingenommen wird.

Aufgrund dieser Gegebenheiten ist es natürlich therapeutisch außerordentlich wünschenswert, nach Mitteln und Wegen zu suchen, die folgendes gewährleisten:

1. Überwachung der Patientenschaft hinsichtlich ihres Verhaltens bei der Einnahme und Dosierung der ihnen vom Arzt verordneten Medikamente.

2. Verbesserung des Verhaltens der Patienten hinsichtlich der Dosierung und Einnahme der Arzneimittel.

3. Minimierung der therapeutischen Konsequenzen aufgrund von Fehlern, die die Patienten bei der Dosierung machen.

4. Minimierung der Wahrscheinlichkeit, daß die behandelnden Ärzte die hinsichtlich Dosierung und Einnahme von Medikamenten auf Seiten der Patienten gemachten Fehler sodann fälschlicherweise interpretieren werden als:

a) Versagen der erwarteten Wirksamkeit des Arzneimittels;

b) Notwendigkeit für die Verordnung einer höheren Dosis als zuvor;

c) Notwendigkeit für die Verordnung eines zusätzlichen Arzneimittels;

d) Notwendigkeit für die Verschreibung eines stärkeren Arzneimittels als das erste;

e) Verschlimmerung oder Verschlechterung des Zustandes des Patienten und/oder

f) Fehldiagnose bezüglich des Zustandes des Patienten.

Die den Patienten unterlaufenen Irrtümer und Fehler hinsichtlich der "Selbstverwaltung" der ihnen verordneten Arzneimittel können natürlich zu einer unnötigen Erhöhung der Kosten, der Zeit, des Leidens sowie des Risikos führen, welchem der Patient bei der Erzielung einer Heilung oder einer Linderung seiner Krankheit ausgesetzt ist. Eine genaue Überwachung und Kontrolle der tatsächlich eingenommenen Arzneimittel würde daher zu einer großen Verbesserung hinsichtlich der Qualität der Therapie führen, die mit den zur Verfügung stehenden Medikationen erreichbar ist.

Gemäß dem Stand der Technik gibt es bereits verschiedene Mittel und Wege, um eine "Selbstverwaltung" der z. B. zur oralen Applikation vorgesehenen Arzneimittel durch den Patienten zu überwachen, jedoch sind diese bekannten Mittel und Wege mit wenigen Ausnahmen sämtlich als indirekt zu bezeichnen, und zwar insofern, als sie nicht den tatsächlichen Beweis erbringen, daß ein verordnetes Medikament in der vorgeschriebenen Dosierung tatsächlich eingenommen worden ist. Darüber hinaus können im Falle, daß verschiedene Stärken der Dosierung für den Patienten verfügbar sind, diese bekannten Maßnahmen nicht aufzeigen, in welcher Dosierungsstärke das Medikament vom Patienten eingenommen worden ist.

Die derzeit verfügbaren Methoden und Techniken zur Überwachung der oralen Arzneimittel-Einnahme können in zwei Kategorien eingeordnet werden:

a) Die kontinuierliche Kategorie,
b) die intermittierende Kategorie.

In der kontinuierlichen Kategorie gibt es bereits eine Anzahl von Anwendungen von elektrischen und mechanischen Mitteln, die zu der Feststellung oder zum Ergreifen geeigneter Maßnahmen dienen, wenn eine orale Applikationsform, z. B. Pille, Tablette oder Kapsel, aus ihrer zugehörigen Packung entnommen worden ist. Hierbei ist "unter geeigneten Maßnahmen" folgendes zu verstehen:

Die Aufzeichnung der Zeit, die Auslösung eines Alarmes oder das Aussenden eines Signales zu einem etwa entfernt angeordneten Überwachungsgerät oder zu einer sich entfernt aufhaltenden dritten Person.

Durch keine der vorstehend erläuterten Methoden kann jedoch tatsächlich festgestellt oder bestätigt werden, in welcher Stärke oder Dosierung ein Medikament, wenn überhaupt von dem Patienten eingenommen worden ist.

Ferner sind in der intermittierenden Kategorie verschiedene Maßnahmen zur Feststellung oder Ermittlung von Dosierungsfehlern bekannt. Solche Maßnahmen bestehen beispielsweise darin, intermittierend Proben vom Blut, vom Urin oder von anderen Körperflüssigkeiten des Patienten zu entnehmen und diese Proben zu analysieren, um auf direktem Wege entweder das Arzneimittel selbst oder einen Metabolit des Arzneimittels oder ganz bestimmte Chemikalien zu ermitteln, deren Vorhandensein eindeutig beweisen, daß das betreffende Arzneimittel von dem Patienten eingenommen worden ist.

Mit Hilfe derartiger Maßnahmen und Methoden läßt sich feststellen, daß das betreffende Arzneimittel tatsächlich eingenommen worden ist und zwar zu irgendeiner gewissen Zeit und in irgendeiner gewissen Dosis, es läßt sich aber damit nicht genau spezifizieren, "wann" das Medikament oder "wieviel" von dem Medikament eingenommen worden ist. Somit sind diese bekannten Methoden mit den Nachteilen behaftet, daß sie sich nicht zu einer Extrapolation von gelegentlichen Messungen oder Ermittlungen zu einer kontinuierlichen Aufzeichnung darüber eignen, wann das Präparat oder Arzneimittel tatsächlich eingenommen worden ist.

Eine weitere bekannte Methode zur Feststellung, ob ein Patent vergessen hat, ein Medikament in der vorgeschriebenen Dosis einzunehmen, besteht darin, das Öffnen und Schließen der Arzneimittelverpackung oder die Entnahme einer Medikamentendosis aus der Verpackung durch den Patienten zu überwachen, beispielsweise gemäß der DE-PS 33 35 301 oder den DE-OSen 35 30 356 und 35 04 431.

Jedoch kann mit Hilfe dieser bekannten Methoden auch kein Beweis geführt werden, daß die vorgeschriebene Dosis von dem Patienten tatsächlich eingenommen worden ist, sondern damit läßt sich nur feststellen, ob die Arzneimittelverpackung geöffnet oder nicht geöffnet worden war und gegebenenfalls wann sie geöffnet worden war.

Mit Rücksicht auf die im Vorangehenden erläuterten Probleme und Schwierigkeiten hinsichtlich der Überwachung der "Selbstverwaltung" von Arzneimitteln durch die Patienten, und insbesondere hinsichtlich der Kontinuität einer derartigen Überwachung liegt der vorliegenden Erfindung nun die Aufgabe zugrunde, ein pharmazeutisches Präparat der eingangs genannten Art zu schaffen, durch das eine sichere und eindeutige Information darüber gewonnen werden kann, ob das vom Arzt verordnete Präparat oder Arzneimittel tatsächlich vom Patienten eingenommen worden ist oder nicht, eventuell ergänzt durch die zusätzlichen Informationen, in welcher Dosis und/oder zu welchem Zeitpunkt die Einnahme erfolgt ist und/oder wo das Präparat im Bereich des Gastro-Intestinal-Trakts lokalisierbar ist.

Diese Aufgabe wird erfindungsgemäß bei einem pharmazeutischen Präparat oder Arzneimittel, insbesondere zur oralen oder analen Verabreichung, durch die Merkmale des kennzeichnenden Teiles des Patentanspruches 1 gelöst. Vorteilhafte Weiterbildungen der Erfindung ergeben sich aus den Unteransprüchen 2 -21.

Die Erfindung bezieht sich weiterhin auf ein Verfahren zur Herstellung eines pharmazeutischen Präparats (Arzneimittels), insbesondere zur oralen oder analen Verabreichung. Dieses Verfahren ist erfindungsgemäß durch die Merkmale des Anspruchs 22 gekennzeichnet.

Durch die Erfindung werden somit spezielle pharmazeutische Mittel geschaffen, die außer dem oder den erforderlichen Wirkstoffen und dgl. in einem geeigneten Ausmaß, einer geeigneten Größe und einer geeigneten Form zusätzlich wenigstens einen magnetischen Werkstoff enthalten, mit dessen Hilfe eine quantitaive Bestimmung präzise gewährleistet ist, ob ein verordnetes Mittel (gegebenenfalls auch zu welchem Zeitpunkt und gegebenenfalls in welcher Dosis) von einem Patienten eingenommen worden ist oder nicht. Wenn ja, ist darüber hinaus auch eine Lokalisierung des Präparats bzw. des magnetischen Werkstoffs innerhalb des Gastro-Intestinal-Trakts möglich.

Hierzu ist lediglich ein äußeres magnetisches Feld, insbesondere ein magnetisches Wechselfeld erforderlich, das von der elektronichen Überwachungsvorrichtung erzeugt wird, und, wenn der Patient, nachdem er das Arzneimittel in der vorgeschriebenen Dosis eingenommen hat, in den Bereich dieses Magnetfelds gelangt oder sich in diesen begibt oder ihn durchquert, dann werden infolge der magnetischen Eigenschaften des in dem Arzneimittel enthaltenen Werkstoffes und infolge einer hierdurch verursachten Umkehr der magnetischen Polarität des Magnetfeldes spezifische Störungen in diesem Feld erzeugt oder induziert, derart, daß diese Störungen mit Hilfe eines geeigneten Empfängers der Überwachungsvorrichtung detektierbar sind, woraus sich der Beweis für die Einnahme des Medikaments ableiten läßt.

Die elektronische Überwachungsvorrichtung, die eine Einrichtung zur Magnetfelderzeugung und eine Empfangsvorrichtung zur Signalabgabe aufweist, stellt ein gesondertes Gerät dar, das beispielsweise von dem Patienten selbst getragen oder aber in ein Objekt eingebracht werden kann, mit dem der Patient tagtäglich in Berührung kommt, beispielsweise in ein Bett, einen Lieblingsstuhl, einen Tisch, an dem die Mahlzeiten eingenommen werden, einen Automobilsitz, eine Türfüllung des Autos, einen Toilettensitz und dgl. mehr.

Hierbei kann es wünschenswert sein, über mehr als eine Überwachungsvorrichtung in dem Lebensbereich des Patienten zu verfügen, z. B. über eine oder mehrere Vorrichtungen zu Hause oder über eine oder mehrere Vorrichtungen an der Arbeitsstelle und/oder im Auto, jeweils in Abhängigkeit von den Lebensgewohnheiten und dem Lebensstil des betreffenden Patienten. Vor allem aber auch im klinischen Bereich können solche elektronischen Überwachungsvorrichtungen zur Verfügung stehen, insbesondere zur Bedienung durch ärztliches Personal oder Krankenpflegepersonal.

Bei dem erfindungsgemäßen Arzneimittel ist ferner gewährleistet, daß zwischen dem nicht aufgelösten und dem aufgelösten Zustand des Arzneimittels unterschieden werden kann, wobei auf der Eigentümlichkeit von Werkstoffen mit magnetischen Eigenschaften, insbesondere Resonanzeigenschaften aufgebaut wird, daß diese auf ein von außen einwirkendes Magnetfeld in unterschiedlicher Weise in Abhängigkeit davon reagieren, ob sich die Werkstoffe oder die aus ihnen gebildeten Körper in einem magnetisch nicht oder unterschiedlich (z. B. schwach) detektierbaren oder in einem magnetisch detektierbaren (z. B. stark detektierbaren) Zustand befinden (vgl. Ansprüche 2-4). In einem solchen detektierbaren Zustand kommen z. B. die Resonanzeigenschaften des magnetischen Werkstoffs voll zur Geltung. Ein derartiger Zustand wird insbesondere dann erreicht, wenn sich das applizierte Präparat, z. B. innerhalb des Gastro-Intestinal-Trakts (Gi-Trakts) aufgelöst hat. Infolgedessen ist beispielsweise eine Unterscheidung zwischen einem vom Patienten eingenommenen Medikament und einem von diesem in seiner Tasche getragenen Medikament gewährleistet.

Ein beispielsweise in Form eines serpentinenartig zusammengefalteten oder wendelförmig gebogenen oder spulen- oder rollenförmig zusammengefügten Drahtes oder Bandes oder Streifens oder Filmes ausgebildetes magnetisches Material wird nämlich zur Auslösung eines nur sehr kleinen oder überhaupt keines Signales führen, während das im auseinandergefalteten oder auseinandergerollten Zustand befindliche magnetische Material ein verhältnismäßig starkes Signal erzeugen wird, aus dem eindeutig geschlossen werden kann, daß das den ursprünglich zusammengerollten, zusammengefalteten oder sonstwie eng zusammengefügten Materialstreifen oder dgl. enthaltende Präparat sich im Gi-Trakt aufgelöst hat und eine Öffnung dieses Streifens zuläßt.

In bevorzugter Weise besteht der dem Präparat zugesetzte magnetische Werkstoff aus einem weichmagnetischen oder einem amorphen Material, insbesondere aus einem amorphen Metall, wie z. B. Eisen oder Bor oder dgl..

Darüber hinaus kann dieser Werkstoff aber auch aus einer amorphen Metallverbindung bestehen, z. B. aus einer Eisen-Silicium-Bor-Kohlenstoff-Verbindung.

Die amorphen Formen von Eisen, Bor und anderen Metallen besitzen bekanntlich keine Toxizität.

In bevorzugter Weise wird ein magnetischer Werkstoff ausgewählt, dessen Magnetisierungskurve oder Hystereseschleife eine große Barkhausen-Unstetigkeit aufweist. Eine derartige Magnetisierungskurve ist durch bedeutende sprunghafte Wechsel oder durch eine regenerative Umkehr der magnetischen Polarisation gekennzeichnet, wenn das applizierte Präparat oder Arzneimittel in das von außen einwirkende Magnetfeld eingebracht und den positiven und negativen Amplituden dieses Feldes ausgesetzt wird. Das Ansprechverhalten des in dem Präparat enthaltenen magnetischen Körpers in seinem detektierbaren Zustand auf derartige sprunghafte Umkehrungen der Polarität äußert sich insbesondere in der Erzeugung von starken harmonischen Schwingungen, welche leicht von dem Ansprechverhalten anderer magnetischer Objekte unterschieden werden können, die sich ebenfalls innerhalb der Reichweite des erzeugten Magnetfeldes befinden können. Die Verwendung von Werkstoffen dieser Art ist z. B. bei elektronischen Diebstahlüberwachungssystemen bekannt (vgl. DE-OS 35 41 536). Zur Aufrechterhaltung seiner magnetischen Eigenschaften im detektierbaren Zustand kann der Werkstoff eine solche Zusammensetzung aufweisen, daß er durch die Verdauungssäfte oder -fermente nicht löslich bzw. nicht angreifbar ist, oder aber, er kann mit einer durch die Verdauungssäfte oder -fermente nicht löslichen bzw. nicht angreifbaren Schutzschicht überzogen sein. Aufgrund dieser Merkmale ist gewährleistet, daß der magnetische Werkstoff entweder mit oder ohne Schutzschicht insbesondere biologisch inert, vor allem aber ungiftig ist.

In bevorzugter Weise kann der aus dem magnetischen Werkstoff gebildete Körper in die jeweilige Verabreichungsform des Präparats, wie z. Pille, Pastille, Tablette, film- oder membranüberzogene Tablette oder Kapsel, Dragée, Zäpfchen oder dgl. entweder eingebettet oder in einen Überzug dieser jeweiligen Verabreichungsform eingebracht sein.

Ferner können in vorteilhafter Weise in Abhängigkeit von der spezifischen Art des Präparats ausgewählte magnetische Werkstoffe mit unterschiedlichen Eigenschaften und/oder mit unterschiedlichen geometrischen Formgebungen vorgesehen sein, wobei von diesen Werkstoffen jeweils mindestens einer einem ganz bestimmten Präparat oder dessen speziellen Applikationsformen zugesetzt ist. Infolgedessen besteht die Möglichkeit, entsprechend unterschiedliche Signale zu detektieren und damit verschiedene Präparate oder Medikamente von einander zu unterscheiden, d.h. also unterschiedliche Medikamente im Körper des betreffenden Patienten nachzuweisen. Darüber hinaus können aber auch bei Verwendung einer Anzahl von magnetischen Werkstoffen mit jeweils unterschiedlichen Eigenschaften bei ein und dem selben Präparat oder Medikament unterschiedliche Dosen von diesem Medikament im Körper des Patienten nachgewiesen werden, was beispielsweise dann von Wichtigkeit ist, wenn eine Sequenz von einer aufbauenden oder einer abnehmenden Dosierungskurve nachvollzogen werden soll. Durch Bildung entsprechender Kombinationen können dann aber auch sowohl unterschiedliche Medikamente und unterschiedliche Dosen des jeweils selben Medikaments im Körper des Patienten nachgewiesen und damit von einander unterschieden werden.

Beispielsweise können somit zwei oder drei verschiedene Arzneidosen im GI-Trakt von einander dadurch unterschieden werden, daß ihre jeweiligen unterschiedlichen Resonanzsignale selektiv detektierbar sind. Da feste Objekte eine Zeitspanne von 12 Stunden und mehr erfordern, um durch den GI-Trakt hindurchzugehen, kann es zu irgendeinem Augenblick der Überwachung während einer Dauerverordnung "Zweimal täglich" soviel wie sechs Arzneidosen in dem GI-Trakt geben.

Wenn die Arzneimittel-Dosen einzeln, wie z. B. in einer Blister-Verpackung (Blasenpackung) verpackt sind, dann kann zusätzlich der jeweilige Zeitpunkt der Entnahme einer Dosis aus der Packung dokumentiert werden, insbesondere in einer Weise, wie dies in der DE-PS 33 35 301 oder den DE-OSen 35 04 431 und 35 30 356 beschrieben ist. Diese Informationen zuzüglich der späteren Detektierung oder Erfassung der magnetischen Resonanzsignale von jeweils spezifischer Kennzeichnung im Körper des Patienten liefern den Beweis sowohl für den genauen Zeitpunkt als auch für die Verifizierung der beispielsweise oralen Applikation eines Medikaments.

Eine derartige Beweisführung ist besonders entscheidend oder kritisch für gewisse Arzneimittel, bei denen eine gewissenhafte und wahrheitsgetreue Einnahme-Bereitwilligkeit des Patienten für eine wirksame und sichere Therapie wesentlich ist und bei denen objektive Daten bezüglich der "Verwaltung" der verordneten Arzneimittel für die Beurteilung des Arztes hinsichtlich der Angemessenheit der Dosis und hinsichtlich des Grades der Arzneimittelwirkung, die sich durch die tatsächlich eingenommene Dosis einstellt, sehr wichtig sind.

Die Bewegungen und Kräfte des menschlichen GI-Traktes sind bekanntlich vielfältig und unterschiedlich und insbesondere dazu fähig, unverdauliche Stoffe zu verdichten, zu verfestigen und zusammenzudrücken, in gleicher Weise aber auch, diese Stoffe zu verteilen oder zu dispergieren.

Das letztere ist in typischer Weise eine Funktion des Magens und des Dünndarmes, das erste in typischer Weise eine Funktion des distalen Kolons (Grimmdarmes). Es ist daher gemäß einer Weiterbildung der Erfindung von Vorteil, wenn der aus dem jeweiligen magnetischen Werkstoff gebildete, in dem zugeordneten Präparat enthaltene Körper ein gewisses Formerinnerungsvermögen (Memory) für seine magnetisch detektierbare, z. B. geöffnete oder auseinandergefaltete oder auseinandergerollte Konfiguration besitzt, so daß z. B. einige Stunden der Aufrechterhaltung dieses magnetisch detektierbaren Zustandes und damit eine entsprechende Gelegenheit für eine genaue und sichere Detektierung gewährleistet werden können. Vorzugsweise ist dieses Formerinnerungsvermögen voreinstellbar.

Ein zu starkes Formerinnerungsvermögen birgt jedoch die Gefahr in sich, daß der aus dem magnetischen Werkstoff gebildete Körper zu unnachgiebig, d.h. nicht flexibel genug ist, um sicher durch den GI-Trakt hindurchzugehen, ohne die Gefahr einer Obstruktion, einer Abrasion oder selbst einer Perforation hervorzurufen. Dieser Gefahr läßt sich durch entsprechende Ausgestaltung des magnetischen Körpers vorbeugen, z. B. dadurch, daß an den jeweiligen Enden des Körpers, z. B. Drahtes, Bandes oder Streifens, abgerundete oder ähnlich geformte Schutzkörper angebracht sind. Beispielsweise kann hierbei auch der magnetische Werkstoff in Form eines Streifens oder Drahtes aus metallischem Glas mit Schutzkörpern an seinen Enden ausgebildet sein.

Gemäß einer weiteren Ausgestaltung der Erfindung kann es vorteilhaft sein, den aus dem magnetischen Werkstoff gebildeten Körper in einer löslichen Umhüllung ganz oder teilweise einzuschließen und den Körper mit seiner Umhüllung dem Präparat zuzusetzen. Eine solche Umhüllung kann vorzugsweise aus einem Material oder mehreren, vorwählbaren Materialien mit jeweils bereichsspezifisch unterschiedlicher Auflösbarkeit im GI-Trakt bestehen, z. B. mit einer spezifischen Auflösbarkeit entweder nur im Magen oder nur im Dünndarm oder nur im Grimmdarm usw. Da der Körper solange magnetisch nicht detektierbar oder völlig andersartig detektierbar ist, als er noch in seiner Umhüllung eingeschlossen ist, d.h., solange sich diese Umhüllung noch nicht aufgelöst hat, ergibt sich in diesem Falle auch die Möglichkeit, mit der magnetischen Detektierbarkeit des Körpers seine Lokalisierbarkeit im Verlaufe seiner Wanderung durch den GI-Trakt zu verbinden.

Darüber hinaus kann das Präparat nach der Erfindung bei Bedarf in der Verabreichungsform einer zweige teilten Kapsel ausgebildet sein, derart, daß der eine Kapselteil den oder die Wirkstoffsubstanzen des Präparats enthält, während im anderen Kapselteil der magnetische Werkstoff oder der aus diesem gebildete Körper untergebracht ist.

Ferner kann der magnetische Wirkstoff oder der aus diesem gebildete Körper zusätzlich noch auf einem Substrat angeordnet sein.

Schließlich besteht noch die Möglichkeit, daß der magnetische Werkstoff oder der aus diesem gebildete Körper von einem durch Flüssigkeit aufquellbaren Material, insbesondere von einem wasserquellbaren Polymer umgeben ist. Durch ein derartiges aufquellbares Material wird praktisch eine Art von Tampon geschaffen, der den magnetischen Werkstoff oder Körper umgibt, wodurch gewährleistet ist, daß dieser Körper bei seiner Wanderung durch den GI-Trakt keinerlei Verletzungen hervorrufen kann. Ein derartiger Tampon kann insbesondere so ausgebildet sein, daß er zunächst als lose Hülle den magnetischen Körper in dessem ersten, noch aufgerollten Zustand umgibt, während er im nächsten Schritt den sodann zusammengerollten Körper entsprechend zusammengeknüllt umgibt, um möglichst wenig Volumen einzunehmen. Nach der Applikation und in dem Augenblick, in welchem der magnetische Körper nach der Auflösung des applizierten Präparates in den magnetisch detektierbaren Zustand übergeführt wird, z. B. die Spule oder Rolle zu einem ausgestreckten oder entrollten Körper verändert wird, dann kann die aufquellbare Materialbeschichtung, insbesondere die wasserquellbare Polymerhülle, durch Aufsaugen der umgebenden Flüssigkeit aufquellen, so daß ein vorzugsweise langgestreckter Schutzkörper oder Schutz-Tampon entsteht, in dessen Innerem der magnetische Werkstoff oder Körper z. B. in Form eines Bandes oder Streifens enthalten ist.

Zur näheren Erläuterung der Erfindung, ihrer weiteren Merkmale und Vorteile dient die beigefügte Beschreibung von Ausführungsbeispielen in Verbindung mit den Zeichnungen.

In diesen zeigt:

Fig. 1 ein Stück eines Drahtes aus einem magnetischen amorphen Material;

Fig. 2 ein Stück eines Bandes oder Streifens aus einem magnetischen amorphen Material;

Fig. 3 einen Schnitt durch ein pharmazeutisches Präparat oder Medikament in Form einer Pille;

Fig. 4 einen Schnitt durch ein Medikament in Form einer Tablette;

Fig. 5 einen Schnitt durch ein Medikament in Form einer Kapsel;

Fig. 6 einen Schnitt durch ein Medikament in Form eines Dragées;

Fig.7 und 8 jeweils einen Schnitt durch aus magnetischem Material gebildete Körper verschiedener Ausführungsarten;

Fig. 9 einen Schnitt durch ein weiteres Medikament in einer weiteren kapselförmigen Ausführung;

Fig. 10 eine Hystereseschleife zur Erläuterung der magnetischen Eigenschaften des den Medikamenten zugesetzten Werkstoffes;

Fig. 11 einen Schnitt durch ein weiteres Medikament in Form einer Pille;

Fig. 12 eine Teil-Schnittansicht eines weiteren, aus magnetischem Werkstoff gebildeten Körpers;

Fig. 13a und 13b jeweils eine Teil-Schnittansicht durch einen aus magnetischem Werkstoff bestehenden Körper gemäß einer weiteren Ausführungsart; und

Fig. 14 eine Seitenansicht eines Medikaments in der Form eines Zäpfchens.

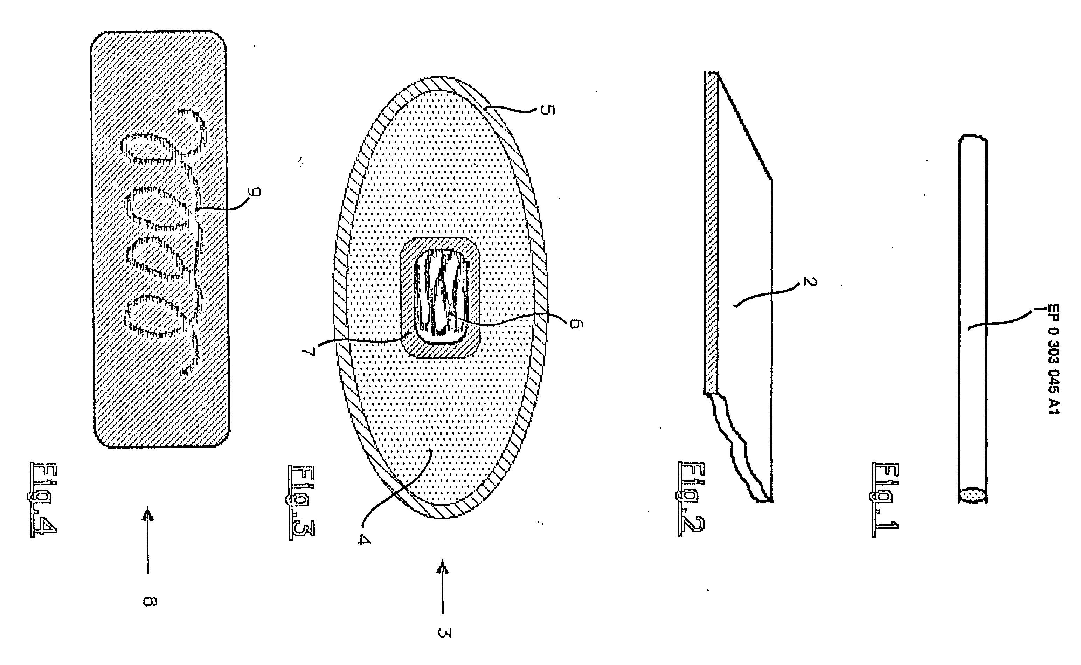

Die in den Fig. 1 - 9 und 11 - 14 dargestellten Ausführungsbeispiele sind jeweils in vergrößerter Darstellung gezeichnet, um das Verständnis der vorliegenden Erfindung zu erleichtern.

Aus der Fig. 1 ist beispielsweise ein Stück eines Drahtes 1 zu ersehen, der aus einem amorphen Metall oder aus einer amorphen Metallverbindung besteht, d.h. also aus einem Werkstoff mit solchen magnetischen Eigenschaften,daß er in der Lage ist, auf ein von außen einwirkendes Magnetfeld zu reagieren, welches z. B. von einer (nicht dargestellten) Überwachungsvorrichtung erzeugt wird.

Wie aus der Fig. 2 ersichtlich, kann aus dem gleichen Werkstoff, wie er zur Bildung eines Drahtes gemäß Fig. 1 dient, aber auch ein Element in Form eines Bandes oder Streifens 2 gebildet sein, wobei auch die Möglichkeit besteht, die Dickendimension eines derartigen Streifens oder Bandes so zu minimieren, daß ein Element in der Form einer Folie oder eines Filmes entsteht.

Die weitere Verwendung des in Fig. 1 dargestellten Drahtes oder des in Fig. 2 dargestellten Bandes oder Streifens in Verbindung mit pharmazeutischen Präparaten wird anhand der folgenden Figuren näher erläutert.

Fig. 3 zeigt eine Schnittansicht durch eine Pille 3, die in üblicher Weise aus einer oder mehreren Wirkstoffen 4 besteht, die nach außen hin durch einen Überzug 5 bedeckt sind.

Die im Inneren dieser Pille 3, d.h. also innerhalb der Wirkstoffe 4, ist nun zusätzlich noch ein Körper 6 eingebettet, der aus einem in etwa serpentinenartig mehr oder weniger eng zusammengefalteten Draht 1 gemäß Fig. 1 oder einem Band oder Streifen 2 gemäß Fig. 2 gebildet ist. Wie die Fig. 3 weiterhin zeigt, kann dieser Körper 6 innerhalb einer Umhüllung 7 von z. B. rechteckförmigem Querschnitt angeordnet sein, wobei diese Umhüllung 7 z. B. aus einem beim Durchgang durch den GI-Trakt löslichen Material, wie etwa Gelatine oder dgl. besteht. Die Umhüllung 7 kann aber auch aus einem Material bestehen, das eine GI-bereichsspezifisch unterschiedliche Löslichkeit aufweist, d.h. als Ort der Auflösung ist z. B. entweder der Dünndarm oder Dickdarm vorgegeben.

Die Umhüllung 7 kann ferner auch so ausgebildet sein, daß sie nur teilweise den Körper 6 umgibt.

Fig. 4 zeigt eine Schnittansicht durch ein Medikament in der Verabreichungsform als Tablette 8, in deren Innerem ein Körper 9 eingebettet ist, der aus einem wendelförmig gebogenen Draht 1 gem. Fig. 1 besteht.

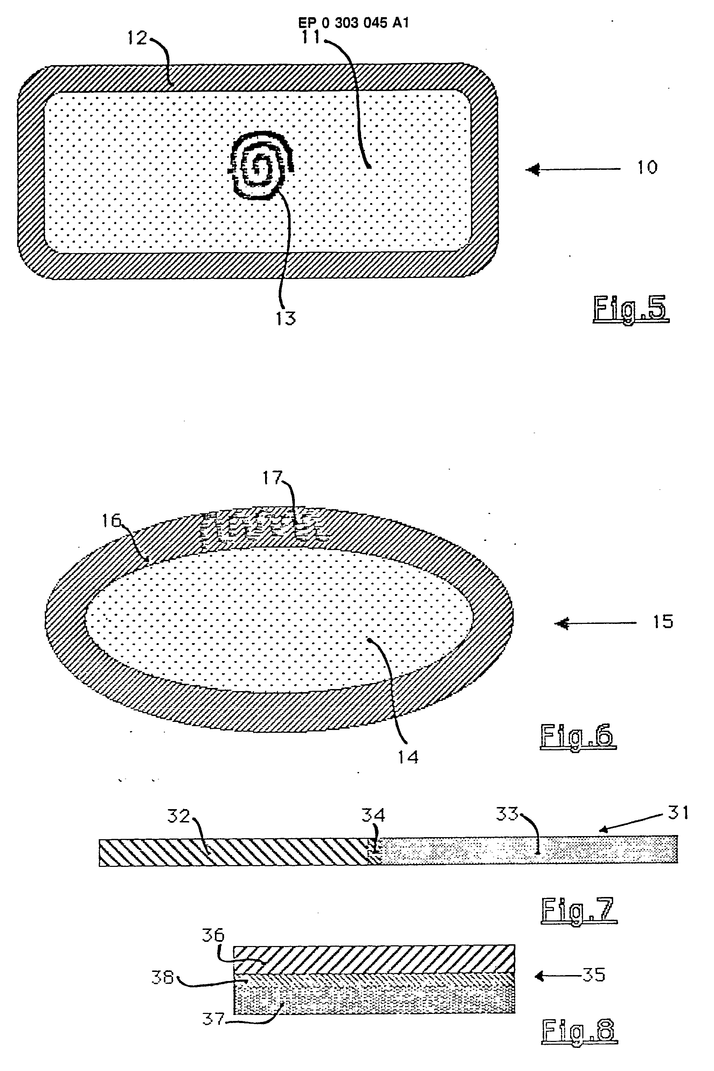

Aus der Fig. 5 ist eine Schnittansicht durch ein weiteres Medikament ersichtlich, das in der Form einer Kapsel 10 ausgebildet ist, bei der innerhalb einer Umhüllung 12, die z. B. aus Gelatine besteht, ein Wirkstoff oder eine Kombination von Wirksubstanzen 11 untergebracht ist. Weiterhin ist im Inneren der Kapsel 10 innerhalb der Wirksubstanzen 11 ein Körper 13 eingebettet, der aus einem spulenförmig oder rollen förmig zusammengefügten Band oder Streifen 2 gem. Fig. 2 gebildet ist.

In der Fig. 6 ist in Schnittansicht ein Dragée 15 dargestellt, bei dem der Wirkstoff oder die Wirkstoffe 14 nach außen hin von einem verhältnismäßig dicken Überzug 16 bedeckt sind.

In diesem Falle besteht die Möglichkeit, einen magnetischen Körper 17, der ähnlich wie der Körper 6 gem. Fig. 3 aus einem serpentinenartig zusammengebogenen oder zusammengelegten Draht 1 gem. Fig. 1 bestehen kann, innerhalb des Überzuges 16 des Dragées 15 unterzubringen.

Weiterhin zeigt die Fig. 9 eine Schnittansicht durch ein Medikament, das ähnlich, wie in Fig. 5 dargestellt, die Gestalt oder äußere Formgebung einer Kapsel aufweist. Bei dem Ausführungsbeispiel gem. Fig. 9 ist jedoch der Kapselinhalt in ein erstes Teil 18a und ein zweites Teil 18b unterteilt, wobei diese beiden Teile 18a und 18b nach außen hin gemeinsam von einer Kapsel-Hülle 21 umschlossen sind.

Der erste Teil 18a des Kapselinhaltes enthält hierbei einen Wirkstoff oder Wirkstoffe 19, während in dem zweiten Teil 18b des Kapselinhalts ein magnetischer Körper 22 untergebracht ist, der in ähnlicher Weise wie der Körper 13 gemäß Fig. 5 ausgebildet ist.

Vorzugsweise befindet sich der Körper 22 gemäß Fig. 9 innerhalb eines Einbettungsmediums 20, welches praktisch gemeinsam mit einer Umhüllung, die etwa ähnlich der Umhüllung 7 gem. Fig. 3 ausgebildet ist, den zweiten Teil 18b des Inhalts der Kapsel 18 bildet, wobei dieses Medium 20 insbesondere biologisch inert und ungiftig ist.

In ähnlicher Weise kann auch für die Körper 9 (gemäß Fig. 4) bzw. 13 (gemäß Fig. 5) bzw. 17 (gemäß Fig. 6) eine Umhüllung 7, wie anhand der Fig. 3 erläutert, vorgesehen sein.

Es versteht sich, daß für die Körper 6 (gem. Fig. 3) bzw. 9 (gemäß Fig. 4) bzw. 13 (gemäß Fig. 5) bzw. 17 (gemäß Fig. 6) bzw. 22 (gemäß Fig. 9) verschiedene magnetische Werkstoffe mit entsprechend unterschiedlichen magnetischen Eigenschaften vorgesehen sein können. Hierbei sind die verschiedensten Kombinationen von Werkstoffen/Werkstoffeigenschaften mit geometrischen Formen der Körper der unterschiedlichsten Art möglich.

Sämtlichen Körpern 6, 9, 13, 17 bzw. 22 ist jedoch gemeinsam, daß sie nach der Auflösung des zugehörigen Präparats oder Medikaments im GI-Trakt in einen solchen geöffneten Ausgangszustand übergeführt oder zurückgeführt werden, in welchem die genannten Körper wiederum die Gestalt eines ausgestreckten Drahtes oder Streifens oder Bandes oder Filmes annehmen, so daß die genannten Körper erst in diesem magnetisch detekierbaren Zustand oder in dieser Konfiguration die erwünschten magne tischen Resonanzeigenschaften zeigen, wodurch die Detektierbarkeit dieser Körper mit Hilfe eines von außen einwirkenden Magnetfeldes gewährleistet ist, wie dies bereits weiter oben ausführlich erläutert wurde.

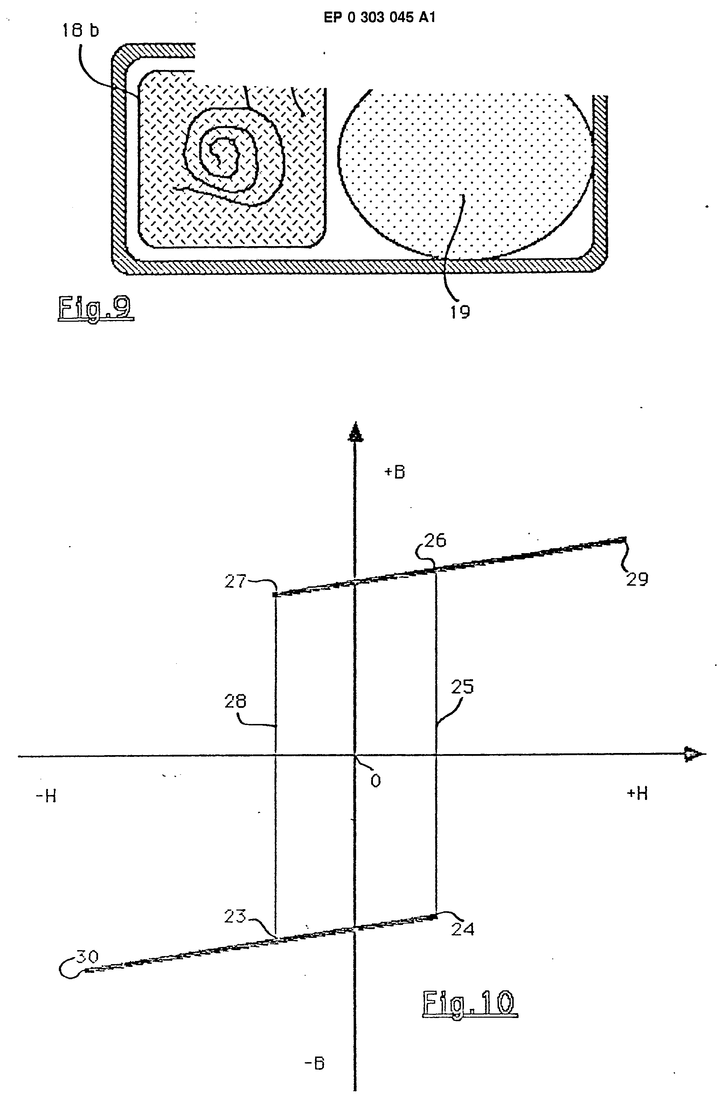

Die dem pharmazeutischen Präparat (Arzneimittel, Medikament) zugesetzten magnetischen Werkstoffe bzw. die aus diesen gebildeten Körper weisen in bevorzugter Weise eine Magnetisierungskurve auf, wie sie beispielsweise in der Fig. 10 dargestellt ist. Dieses Diagramm enthält aus Gründen der Übersichtlichkeit eine vereinfacht gezeichnete Hystereseschleife, mit der magnetischen Feldstärke H auf der Abszisse und der magnetischen Induktion B auf der Ordinate.

H stellt somit das von außen einwirkende magnetische Feld dar.

Der negative maximale Induktionspunkt (-B$_{max}$) ist mit 23, der positive Schwellwert des magnetischen Feldes mit 24, der positive maximale Induktionspunkt (+B$_{max}$) mit 26 und der negative Schwellwert des magnetischen Feldes mit 27 bezeichnet. Wenn das magnetische Feld den Punkt 24 erreicht und diesen überschreitet, kommt es zu einer abrupten regenerativen Umkehr der Polarität, so daß auf der Hystereseschleife der Punkt 26 (+B$_{max}$) erreicht wird. Die Linie 25 zwischen den Punkten 24 und 26 stellt somit einen großen Barkhausen-Sprung dar, gemäß dem die Polaritätsumkehrung erfolgt.

Wenn auf der anderen Seite das magnetische Feld H in umgekehrter Richtung den Nullpunkt durchläuft und weiter in negativer Richtung geht, dann wird schließlich der Punkt 27, d.h. der negative Schwellwert des magnetischen Feldes erreicht, von dem aus wiederum eine abrupte regenerative Umkehr der Polarität stattfindet, bis der Punkt 23 (-B$_{max}$) erreicht ist. Die Linie 28 zwischen den Punkten 27 und 23 stellt wiederum den erneuten großen Barkhausen-Sprung in diesem Bereich dar.

Auf der Magnetisierungskurve gemäß Fig. 10 sind ferner die Punkte positiver Sättigung bzw. negativer Sättigung mit 29 bzw. 30 bezeichnet.

Selbstverständlich ist die für das jeweilige magnetische Material kennzeichnende Hystereseschleife abhängig von der speziellen Werkstoffzusammensetzung und den jeweiligen geometrischen Formen und Abmessungen des hieraus gebildeten Körpes.

Wie sich aus den Fig. 7 oder 8 ersehen läßt, besteht auch die Möglichkeit, zunächst einen ersten Verbundkörper 31 oder einen zweiten Verbundkörper 35 zu bilden, welche jeweils z. B. einem der in den Fig. 3 - 6 bzw. 9 dargestellten Medikamente anstelle der dort gezeigten magnetischen Körper zugesetzt werden können.

In dem aus den Fig. 7 oder 8 ersichtlichen Zustand befinden sich die Verbundkörper 31 oder 35 in einem jeweils magnetisch nicht detektierbaren Zustand, und zwar dadurch, daß ein erster Teilkörper 32 aus einem weichmagnetischen Material mit einem zweiten Teilkörper 33 aus einem Dauermagnetwerkstoff (Hartmagnet) mittels einer Verbindungsschicht 34 in stirnseitig aufeinander zugewendeten Bereichen zu dem bereits genannten Verbundkörper 31 vereinigt ist (Fig. 7), oder daß - in der Art einer Aufeinanderschichtung - eine erste, platten- oder folienförmige Komponente 36 aus einem weichmagnetischen Material mit einer zweiten, entsprechend geformten Komponente 38 in Form einer Verbindungsschicht zu dem bereits genannten zweiten Verbundkörper 35 zusammengesetzt ist (Fig. 8).

Die Verbindungsschichten 34 bzw. 38 bei den Verbundkörpern 31 bzw. 35 bestehen aus einem jeweils durch die Verdauungssäfte oder -fermente löslichen Material, so daß der jeweilige Verbundkörper 31 bzw. 35 nach einer Auflösung des zugehörigen, einem Patienten verabreichten Präparates in dem GI-Trakt in einen für die elektronische Überwachungsvorrichtung magnetisch detektierbaren Zustand dadurch übergeführt wird, daß der Teilkörper 33 (Fig. 7) bzw. die Komponente 37 (Fig. 8) infolge Auflösung der Schichten 34 bzw. 38 von dem jeweiligen Verbundkörper 31 bzw. 35 abgelöst oder abgetrennt werden.

Infolgedessen stellt sich für den ersten Teilkörper 32 des Verbundkörpers 31 gemäß Fig. 7 bzw. für die erste Komponente 36 des Verbundkörpers 35 gemäß Fig. 8 ein jeweils magnetisch detektierbarer Zustand ein, in welchem die erwünschten magnetischen Eigenschaften des Teilkörpers 32 bzw. der Komponente 36 voll zur Geltung gelangen, so daß beispielsweise mit Hilfe eines äußeren magnetischen Wechselfeldes, das von der elektronischen Überwachungs vorrichtung erzeugt wird, die entsprechenden Detektionsmöglichkeiten bezüglich des applizierten Präparates wiederum gegeben sind.

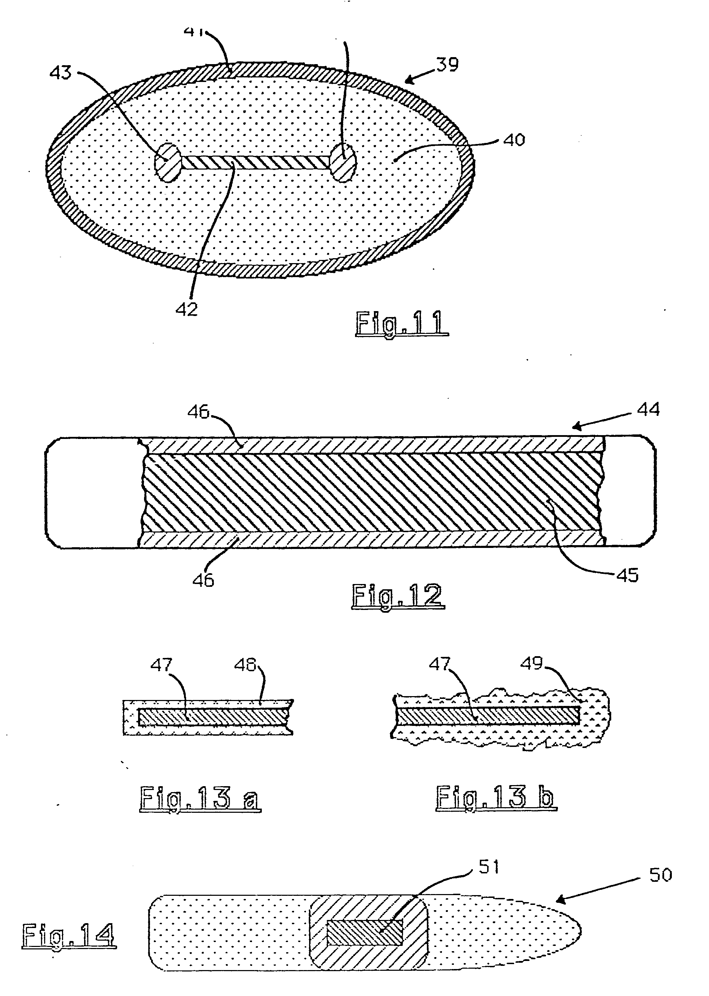

In Fig. 11 ist ein Ausführungsbeispiel eines pharmazeutischen Präparates in der Form einer weiteren Pille 39 dargestellt, die im wesentlichen aus einem den oder die Wirkstoffe 40 enthaltenden inneren Teil und einem auf diesen außen aufgebrachten Überzug 41 besteht. Im inneren Teil der Pille 39 ist wiederum ein aus einem magnetischen Werkstoff gebildeter, sechster Körper 42 untergebracht, welcher im vorliegenden Ausführungsbeispiel die Gestalt eines kurzen, ausgestreckten Drahtes, Bandes oder Streifens besitzt. An den Enden des Körpers 42 befinden sich jeweils kugelförmig oder ähnlich ausgeformte Schutzkörper 43 aus einem geeigneten, beispielsweise elastisch deformierbaren und ansonsten inerten Material, derart, daß sich nach Auflösung des applizierten Präparates im GI-Trakt durch den Körper 42 aus magnetischem Material keinerlei Verletzungsgefahren im Bereich der Magen- oder Darmwände ergeben.

Ferner ist aus Fig. 12 ein Ausführungsbeispiel für einen weiteren, aus einem magnetischen Werkstoff 45 gebildeten, siebten Körper 44 ersichtlich, dessen Oberflächen mit einer durch die Verdauungssäfte oder -fermente nicht löslichen bzw. nicht angreifbaren Schutzschicht 46 beschichtet sind, so daß der gesamte Körper 44 biologisch inert und ungiftig ist.

Stattdessen könnte auch ein magnetischer Werkstoff 45 mit einer solchen Zusammensetzung ausgewählt werden, daß er durch die Verdauungssäfte nicht löslich bzw. nicht angreifbar ist und im übrigen biologisch inert, vor allem ungiftig ist.

Fig. 13a zeigt eine Schnittansicht durch einen weiteren, teilweise dargestellten Körper aus einem magnetischen Werkstoff 47, dessen Oberfläche von einer wasseraufquellbaren Polymerhülle 48 umgeben ist. Nach Auflösung des applizierten, den Werkstoff 47 enthaltenden Medikaments im GI-Trakt des Patienten, stellt sich ein Zustand ein, der aus der Fig. 13b ersichtlich ist und in dem sich die ursprüngliche Polymerhülle 48 in eine durch Flüssigkeit aufgequollene Hülle 49 verwandelt hat, so daß ein quasi langgestreckter Tampon aus dem magnetischen Werkstoff entsteht, derart, daß infolge der Applikation keinerlei Verletzungsgefahren im inneren Bereich des Magen-Darmtraktes auftreten können.

Schließlich zeigt die Fig. 14 noch ein Ausführungsbeispiel eines Medikaments in der Form eines Zäpfchens 50, in dessen Innerem ein aus magnetischem Werkstoff gebildeter, achter Körper 51 untergebracht ist, in entsprechender Weise, wie dies bereits anhand der Ausführungsbeispiele gemäß den Fig. 3 - 6, 9 bzw. 11 erläutert worden ist.

### Bezugszeichenliste

1 Draht
2 Band (Streifen, Film)
3 Pille
4 Wirkstoff
5 Überzug
6 Erster Körper (aus einem serpentinenartig zusammengefalteten Draht 1 oder Band 2)
7 Umhüllung
8 Tablette
9 Zweiter Körper (aus einem wendelförmig gebogenen Draht 1)
10 Kapsel
11 Wirkstoff
12 Umhüllung (von 11)
13 Dritter Körper (aus einem spulenförmig zusammengefügten Band)
14 Wirkstoff
15 Dragée
16 Überzug
17 Vierter Körper
18 Kapsel
18a Erster Teil des Kapselinhalts
18b Zweiter Teil des Kapselinhalts
19 Wirkstoff
20 Einbettungsmedium
21 Kapsel-Hülle
22 Fünfter Körper
23 Negativer maximaler Induktionspunkt
24 Positiver Schwellwert des magnetischen Feldes
25 Barkhausen-Sprung
26 Positiver maximaler Induktionspunkt

27 Negativer Schwellwert des magnetischen Feldes
28 Barkhausen-Sprung
29 Punkt positiver Sättigung
30 Punkt negativer Sättigung
31 Erster Verbundkörper
32 Erster Teilkörper
33 Zweiter Teilkörper
34 Verbindungsschicht
35 Zweiter Verbundkörper
36 Erste Komponente (von 35)
37 Zweite Komponente (von 35)
38 Dritte Komponente (Verbindungsschicht)
39 Pille
40 Wirkstoff
41 Überzug
42 Sechster Körper
43 Schutzkörper
44 Siebter Körper
45 Magnetischer Werkstoff
46 Schutzschicht
47 Magnetischer Werkstoff
48 Polymerhülle
49 Aufgequollene Hülle
50 Zäpfchen
51 Achter Körper

### Ansprüche

1. Pharmazeutisches Präparat (Arzneimittel) insbesondere zur oralen oder analen Verabreichung (Applikation),
dadurch gekennzeichnet,
daß dem Präparat, insbesondere dem (den) Wirkstoff(en) des Präparats wenigstens ein mangnetischer Werkstoff zugesetzt ist, der auf ein von außen einwirkendes Magnetfeld einer elektronischen Überwachungsvorrichtung reagierfähig ist, um ein durch diese Überwachungsvorrichtung selektiv detektierbares Signal zur Lieferung einer eindeutigen Information abzugeben, ob das Präparat von einem Patienten eingenommen ist oder nicht, und/oder zur Lokalisierung des Präparats bzw. des magnetischen Werkstoffs im Bereich des Gastro-Intestinal Trakts des Patienten.

2. Präparat nach Anspruch 1,
dadurch gekennzeichnet,
daß aus dem jeweiligen magnetischen Werkstoff ein zunächst magnetisch nicht oder unterschiedlich detektierbarer Körper gebildet ist, der nach Auflösung des applizierten Präparates in dem Gastro-Intestinal-Trakt des Patienten ebenfalls infolge Einwirkung der Verdauungssäfte bzw. -fermente in einen magnetisch detektierbaren Zustand für die elektronische Überwachungsvorrichtung überfuhrbar ist, in welchem Zustand die Eigenschaften, insbesondere Resonanzeigenschaften des magnetischen Werkstoffes zur Geltung kommen.

3. Präparat nach Anspruch 1,
dadurch gekennzeichnet,
daß aus dem jeweiligen magnetischen Werkstoff ein zunächst magnetisch nicht oder unterschiedlich detektierbarer Körper (6; 9; 13) z. B. in der Form eines serpentinenartig zusammengefalteten oder wendelförmig gebogenen oder spulen-oder rollenförmig zusammengefügten Drahtes oder Bandes oder Streifens oder Filmes gebildet ist, derart, daß dieser Körper nach Auflösung des applizierten Präparates in dem Gastro-Intestinal-Trakt des Patienten in einen geöffneten und damit magnetisch detektierbaren z. B. auseinandergefalteten oder auseinandergerollten Zustand für die elektronische Überwachungsvor richtung überführbar ist, in welchem Zustand die Eigenschaften, insbesondere Resonanzeigenschaften des magnetischen Werkstoffes zur Geltung kommen.

4. Präparat nach Anspruch 1,
dadurch gekennzeichnet,
daß aus dem jeweiligen magnetischen Werkstoff ein zunächst magnetisch nicht oder unterschiedlich detektierbarer Körper dadurch gebildet ist, daß der magnetische Körper mit mindestens einem weiteren mit ihm einen Verbundkörper bildenden Teilkörper lösbar verbunden ist, und daß dieser magnetische Körper nach Auflösung des applizierten Präparates in dem Gastro-Intestinal-Trakt des Patienten in einen für die elektronische Überwachungsvorrichtung magnetisch detektierbaren Zustand dadurch überführbar ist, daß dieser Teilkörper infolge der Einwirkung der Verdauungssäfte oder -fermente von dem Verbundkörper lösbar oder abtrennbar ist, wobei in dem detektierbaren Zustand die Eigenschaften, insbesondere Resonanzeigenschaften des magnetischen Werkstoffes zur Geltung kommen.

5. Präparat nach einem der Ansprüche 1 - 4,
dadurch gekennzeichnet,
daß der Werkstoff aus einem weichmagnetischen Material besteht.

6. Präparat nach einem der Ansprüche 1 - 5,
dadurch gekennzeichnet,
daß der Werkstoff aus einem amorphen Material besteht.

7. Präparat nach Anspruch 6,
dadurch gekennzeichnet,
daß der Werkstoff aus einem amorphen Metall, z. B. Eisen, Bor oder dgl., besteht.

8. Präparat nach Anspruch 6,
dadurch gekennzeichnet,
daß der Werkstoff aus einer amorphen Metallverbindung besteht. z. B. aus einer Eisen-Silicium-Bor-Kohlenstoff-Verbindung.

9. Präparat nach einem der vorangehenden Ansprüche,
dadurch gekennzeichnet,
daß der Werkstoff eine Magnetisierungskurve mit einer großen Barkhausen-Unstetigkeit aufweist.

10. Präparat nach einem der vorangehenden Ansprüche,
dadurch gekennzeichnet,
daß der magnetische Werkstoff eine solche Zusammensetzung aufweist, daß er durch die Verdauungssäfte oder -fermente nichtlöslich bzw. nichtangreifbar ist, oder mit einer durch die Verdauungssäfte oder -fermente nichtlöslichen bzw. nichtangreifbaren Schutzschicht überzogen ist. so daß der Werkstoff mit oder ohne Schutzschicht insbesondere biologisch inert, vor allem ungiftig ist.

11. Präparat nach einem der vorangehenden Ansprüche,
dadurch gekennzeichnet,
daß der Werkstoff oder der aus dem Werkstoff gebildete Körper in die jeweilige Verabreichungsform des Präparats (Pille, Pastille, Tablette, Kapsel, film- oder membranüberzogene Tablette oder Kapsel, Dragée, Zäpfchen oder dgl.) eingebettet ist.

12. Präparat nach einem der vorangehenden Ansprüche
dadurch gekennzeichnet,
daß der Werkstoff oder der aus dem Werkstoff gebildete Körper (17) in einen Überzug (16) der jeweiligen Verabreichungsform des Präparats eingebracht ist.

13. Präparat nach einem der vorangehenden Ansprüche,
dadurch gekennzeichnet,
daß in Abhängigkeit von der spezifischen Art des Präparats ausgewählte magnetische Werkstoffe mit unterschiedlichen Eigenschaften und/oder mit unterschiedlichen geometrischen Formgebungen vorgesehen sind, von denen jeweils mindestens einer einem bestimmten Präparat bzw. dessen Applikationsformen zugesetzt ist, wodurch entsprechend unterschiedliche Signale detektierbar sind.

14. Präparat nach einem der Ansprüche 2 - 13,
dadurch gekennzeichnet,
daß der aus dem magnetischen Werkstoff gebildete Körper ein Formerinnerungsvermögen (Memory) für seine magnetisch detektierbare, z. B. geöffnete oder auseinandergefaltete oder auseinandergerollte Konfiguration besitzt.

15. Präparat nach Anspruch 14,
dadurch gekennzeichnet,
daß das Formerinnerungsvermögen (Memory) voreinstellbar ist.

16. Präparat nach einem der Ansprüche 2 - 15,
dadurch gekennzeichnet.
daß der aus dem magnetischen Werkstoff gebildete Körper (6) in einer löslichen Umhüllung (7) ganz oder teilweise eingeschlossen und mit der Umhüllung (7) dem Präparat zugesetzt ist, wobei diese Umhüllung aus einem oder mehreren, vorwählbaren Materialien mit bereichsspezifisch unterschiedlicher Auflösbarkeit im GI-Trakt, z. B. entweder nur im Magen oder nur im Dünndarm oder nur im Grimmdarm usw., besteht.

17. Präparat nach einem der vorangehenden Ansprüche
dadurch gekennzeichnet,
daß es in der Verabreichungsform einer zweigeteilten Kapsel (18) ausgebildet ist, wobei ein Kapselteil den oder die Wirkstoffsubstanzen (19) und der andere Kapselteil den magnetischen Werkstoff oder den aus diesem gebildeten Körper (22) enthalten.

18. Präparat nach einem der vorangehenden Ansprüche,
dadurch gekennzeichnet,
daß der magnetische Werkstoff oder der aus diesem gebildete Körper auf einem Substrat angeordnet ist.

19. Präparat nach einem der Ansprüche 2 - 18,
dadurch gekennzeichnet,
daß an den jeweiligen Enden des aus dem magnetischen Werkstoff gebildeten, in das Präparat eingebrachten Körpers, z. B. des Drahtes oder Bandes oder Streifens, Schutzkörper, z. B. abgerundete oder ähnlich geformte Schutzkörper, angebracht sind.

20. Präparat nach einem der vorangehenden Ansprüche,
dadurch gekennzeichnet,
daß der magnetische Werkstoff in der Form eines Streifens oder Drahtes aus metallischem Glas ausgebildet ist, wobei an den jeweiligen Enden dieses Streifens oder Drahtes Schutzkörper vorgesehen sind.

21. Präparat nach einem der vorangehenden Ansprüche,
dadurch gekennzeichnet,
daß der magnetische Werkstoff oder der aus diesem gebildete Körper von einem durch Flüssigkeit aufquellbaren Material, insbesondere von einem wasserquellbaren Polymer umgeben ist.

22. Verfahren zur Herstellung eines pharmazeutischen Präparats (Arzneimittels), insbesondere zur oralen oder analen Verabreichung,
dadurch gekennzeichnet,
daß der Zubereitung des Präparates aus dem (den) vorgesehenen Wirkstoff(en) und gegebenenfalls weiteren Zusätzen und/oder Überzügen oder Umhüllungen wenigstens ein magnetischer Werkstoff gemäß einem oder mehreren der Ansprüche 1 - 21 zugesetzt wird.

EP 0 303 045 A1

Fig.1

Fig.2

Fig.3

Fig.4

EP 0 303 045 A1

Fig.5

Fig.6

Fig.7

Fig.8

18 b

Fig.9

19

+B

26

27

29

28

25

-H

0

+H

23

24

30

-B

Fig.10

41

43

39

40

42

<u>Fig.11</u>

46

44

46

45

<u>Fig.12</u>

47 48

47 49

<u>Fig.13 a</u>

<u>Fig.13 b</u>

51

50

<u>Fig.14</u>

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 012 157 (JOHNSON & JOHNSON) * Seite 2, Zeilen 6-10; Seite 3, Zeile 14 - Seite 4, Zeile 5 * | 1-22 | A 61 K 9/00 A 61 B 5/06 |
| D,A | DE-A-3 541 536 (SENSORMATIC ELECTRONICS CORP.) * Ansprüche 1-14 * | 1-22 | |
| A | EP-A-0 000 667 (NORTHWESTERN UNIVERSITY) * Ansprüche 1-4 * | 1-22 | |

**RECHERCHIERTE SACHGEBIETE (Int. Cl.4)**

A 61 K
A 61 B

Der vorliegende Recherchenbericht wurde für alle Patentansprüche erstellt

| Recherchenort | Abschlußdatum der Recherche | Prüfer |
|---|---|---|
| DEN HAAG | 25-10-1988 | MUELLNERS W. |